# EUROPEAN PATENT SPECIFICATION

(11) **EP 3 937 847 B1**
(45) Date of publication and mention of the grant of the patent: **14.10.2026**
(21) Application number: 20710879.6
(22) Date of filing: 06.03.2020
(51) Int. Cl.: A61F 2/08, A61L 27/18, A61L 27/50, A61L 27/56

(54) **TISSUE REPAIR SCAFFOLD AND DEVICE**
GEWEBEREPARATURGERÜST UND VORRICHTUNG
ÉCHAFAUDAGE ET DISPOSITIF DE RÉPARATION TISSULAIRE

(30) Priority: 12.03.2019 GB 201903388
(43) Date of publication of application: 19.01.2022
(73) Proprietor: The University Of Manchester, Manchester, Greater Manchester M13 9PL (GB)
(72) Inventor: CARTMELL, Sarah, Manchester Greater Manchester M13 9PL (GB); BOSWORTH, Lucy, Manchester Greater Manchester M13 9PL (GB); WONG, Jason, Manchester Greater Manchester M13 9PL (GB); MCGROUTHER, Duncan Angus, Manchester Greater Manchester M13 9PL (GB)
(74) Representative: Dehns
(86) International application number: PCT/EP2020/056104
(87) International publication number: WO 2020/182689

(56) References cited:
- WO-A1-2011/031854
- WO-A1-2017/050837
- CN-U- 204 484 405
- US-A1- 2011 264 237
- US-B2- 9 770 529
- GOH J C.H. ET AL: "Regenerative Medicine and Biomaterials for the Repair of Connective Tissues", 31 December 2010 (2010-12-31), pages 1 - 7, XP093052749, Retrieved from the Internet <URL:https://www.sciencedirect.com/topics/engineering/knitted-scaffold> [retrieved on 20230608]
- WINTERMANTEL E ET AL: "Tissue engineering scaffolds using superstructures", BIOMATERIALS, ELSEVIER, AMSTERDAM, NL, vol. 17, no. 2, 1 January 1996 (1996-01-01), pages 83 - 91, XP004032806, ISSN: 0142-9612, DOI: 10.1016/0142-9612(96)85753-X
- PRAJWAL BHASKAR ET AL: "Cell response to sterilized electrospun poly([epsilon]-caprolactone) scaffolds to aid tendon regeneration in vivo : USE OF PCL AS A BIOMATERIAL SCAFFOLD IN TENDON", JOURNAL OF BIOMEDICAL MATERIALS RESEARCH PART A, vol. 105, no. 2, 1 February 2017 (2017-02-01), pages 389 - 397, XP055700160, ISSN: 1549-3296, DOI: 10.1002/jbm.a.35911
- KLOMPMAKER J ET AL: "Porous implants for knee joint meniscus reconstruction: A preliminary study on the role of pore sizes in ingrowth and differentiation of fibrocartilage", CLINICAL MATERIALS, ELSEVIER, GB, vol. 14, no. 1, 1 January 1993 (1993-01-01), pages 1 - 11, XP023261207, ISSN: 0267-6605, [retrieved on 19930101], DOI: 10.1016/0267-6605(93)90041-5

## Description

### Field of the Invention

The present invention relates to a tissue repair scaffold, a tissue repair device and a method of making the tissue repair scaffold. For example, the invention relates to a tissue repair device for treating a damaged tendon.

### Background

Conventional approaches to the repair of tissue damage and assisting in the recovery from such damage are not comprehensive and have a number of drawbacks. Thus, tissue damage, and particularly damage to tendons, represents a significant challenge.

Tendons are a form of connective tissue and possess great flexibility and elasticity, which allow forces generated by muscle contraction to be transmitted to the attached bone, enabling movement. As a result of their ability to absorb external forces, tendons are able to act as a buffer, helping to prevent injury to the attached muscle.

Natural tendon is an example of highly organised hierarchical tissue. It is principally composed of aligned collagen type I fibres with tenocytes arranged in rows between these fibres. Mechanical properties of tendons differ depending on their location within the body. *In vivo* studies on human Achilles [Magnusson *et al.,* 2003] and tibialis anterior [Maganaris, 1999] tendons yielded moduli of 788 MPa and 1.2 GPa and tensile strengths of 36.5 MPa and 25 MPa respectively. However, testing was not performed to rupture and can only be used as a guide.

All tendons have the potential to be affected by direct damage caused by lacerations or other accidental injuries. They are also susceptible to diseases. Clinically, tendon disorders are referred to as a "tendinopathy", as this makes no assumption as to the pathological processes within the tendon, although the term "tendonitis" is still used.

Of particular concern are tendinopathies within the Achilles tendon, which cause degeneration of the tissue. These are often the result of excessive and repetitive over-loading of the Achilles tendon in both sporting and sedentary patients.

Other tendons prone to pathology include the rotator cuff in the shoulder, where degeneration and the size of tears typically increases with age, and the patella tendon in the knee, which experiences degeneration due to excess load bearing and strain rather than inflammatory tendonitis.

A variety of treatments are employed for tendinopathy management. In the early phase of disease, conservative methods (such as the use of non-steroidal anti-inflammatory drugs (NSAIDs) and corticosteroids) are customarily employed. For those patients who do not respond well to these treatments after 6 months, surgical intervention is common.

For patients presenting acute rupture of the Achilles tendon, treatment falls within three main categories: open operative, percutaneous operative and non-operative.

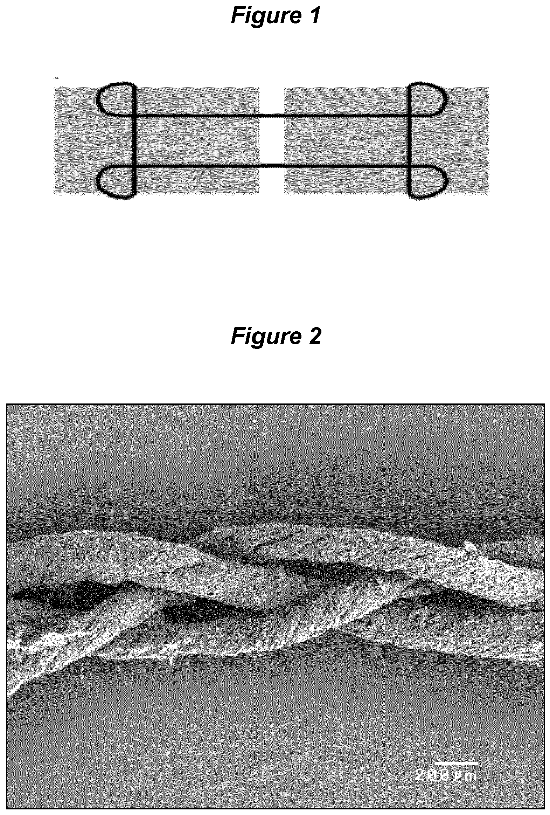

Open operative surgery involves the repair of the two ruptured ends of the tendon by suturing them together. Percutaneous operative is a combination of open and non-operative techniques and involves a number of small incisions used to suture the tendon without fully exposing the tissue. Non-operative treatments involve the immobilisation of the lower leg in a plaster cast for a period of 6-8 weeks. Currently, surgeons often repair tendon lacerations using a suture knot similar to the Kessler knot shown in Figure 1. However, gapping can subsequently occur between the two cut tendon ends, which leads to a poor outcome, including the possibility of the tendon failing to heal.

Following injury, tendon heals by production of scar tissue, which is organisationally, biochemically and biomechanically inferior to normal tendon matrix tissue. Such inferior scar tissue leads to ongoing morbidity of affected patients. Due to the often poor response to the treatment, and resultant morbidity of tendon disease, there is a growing interest in novel techniques for repair of such tissues.

Previous strategies employed to improve the quality of tendon repair after injury include xenograft tendons cross-linked with glutaraldehyde [Smith *et al.,* 1986]. Tissue engineering approaches have utilised autologous tenocytes in biomaterial scaffolds [Cao *et al.,* 2002]. Collagen-based scaffolds have been investigated in an attempt to match the mechanical properties with native tendon [Venugopal *et al.,* 2005 and Curtis *et al.,* 2005].

Synthetic bioresorbable polymers such as polycaprolactone (PCL), polylactic acid (PLA) and chitin have been formed as fibrous mats of randomly orientated fibres, but with limited success [Li *et al.,* 2003]. In WO 2010/067086, a tissue repair scaffold was described, comprising a secondary fibre bundle made of a plurality of primary fibre bundles. Each of these primary fibre bundles comprises a plurality of PCL fibres. One example of a scaffold disclosed in that document involved plaiting a plurality of primary bundles, as shown herein in Figure 2. However, this structure may in some circumstances result in damage to cell ingrowth and tissue in micropores of the rope-like plait, due to a pinching effect (scissoring).

The present invention has been devised in light of the above considerations.

### Summary of the Invention

The term "biocompatible polymer" as used herein will be familiar to the skilled reader but, for completeness, it pertains to a polymer that is compatible with natural tissue, such that a significant immune response or other rejection response is not observed when the polymer is inserted (e.g. surgically implanted) into the human or animal body. The skilled reader will be aware of examples of biocompatible polymers, for example poly-ε-caprolactone (also known as polycaprolactone or PCL).

The terms "biodegradable polymer" and "bioresorbable polymer" as used herein will be familiar to the skilled reader but, for completeness, pertain to a polymer that breaks down and disperses *in vivo.*

The term "tissue repair" as used herein will be familiar to the skilled reader but, for completeness, it pertains to the repair of the natural tissue of a human or animal, for example by replacement or growth (including "regrowth") of that tissue.

The present invention seeks to address the drawbacks discussed above.

The present inventors have found that tissue repair, and in particular tendon repair, can be achieved by providing a tissue repair scaffold having a morphology and/or composition adapted to promote adhesion and growth of tendon cells, thereby facilitating tendon growth.

At its most general, the present invention proposes that a scaffold comprising a knitted body comprising a yarn of a biocompatible polymer provides an effective mimic of natural tissue, and in particular tendons. Another general proposition of the present invention is that a scaffold comprising a knitted body having apertures which remain open, whether the knitted body is loaded or unloaded, is suitable for use in the repair of natural tissues, preferably soft tissues, more preferably connective soft tissues and most preferably tendons. These scaffolds are considered by the present inventors to provide a matrix that suitably not only exhibits appropriate biomechanical properties (particularly for tendon repair) but also facilitates growth of cells within the matrix.

In a first aspect, the present invention provides a tendon repair scaffold as defined in claim 1.

Examples of tendons for which the scaffold of the present invention is particularly effective include: Achilles tendon, biceps brachii, extensor digitorum tendons, extensor indicis, extensor pollicis longus, supraspinatus tendon, tibialis posterior tendons, patella tendon and peroneal tendons.

The present inventors have found that embodiments comprising this knitted body provide excellent biomechanical properties and an environment that promotes growth of tendons, in particular tendon cells such as tenocytes and chondrocytes.

The present inventors have found, through experimentation, that a scaffold which has a knitted body made of a yarn comprising a biocompatible polymer can encourage the growth of tissue cells such as tenocytes. Without wishing to be bound by theory, the present inventors believe that the provision of apertures formed by the interlocking loops of the knitted structure facilitates tissue cell growth.

In particular, as the apertures are open at and between the first configuration in which the knitted body is unloaded (i.e. unstretched) and the second configuration in which the knitted body is loaded (i.e. stretched), this means that the scaffold is tailored to optimise cell-growth under real-world loading. Even under significant strain, the apertures and yarn provide an accommodating environment for cell growth. Although the apertures will change in size depending on the load or tension applied, the apertures remain open, and this functionality has an advantageous effect in promoting cell in-growth development of tissue mass over an extended period of time. The knitted structure permits some variation in length to accommodate movement between the first, unloaded configuration and the second, loaded configuration. Suitably this retention of the open state of the apertures avoids or at least reduces the potential difficulty of "scissoring" in which apertures of openings in a tissue repair scaffold close or become very small and impinge on cells/tissue that has grown into/through the apertures. In turn this may contribute to preventing scar tissue growth.

The knitted body has significant elasticity and flexibility, which facilitates the transfer of load to surrounding tissue. This mimics the inherent elasticity of natural tissue, encouraging cell ingrowth. The variation of length of the knitted body under physiological forces also provides the benefit of allowing an injured patient to undergo physiotherapy, rather than having the injury immobilised.

Whereas a braided or plaited structure may exhibit scissoring, through which cell ingrowth and/or tissue in pores become damaged because of a pinching effect, this is not observed when using the knitted body. Moreover, a knitted structure is inherently less prone to kinking than the rope-like "solid-walled" structure of a braid or plait.

Suitably, the yarn consists essentially of and preferably consists of a biocompatible polymer. Thus, suitably the knitted body and suitably the scaffold consists essentially of, and preferably consists of, a biocompatible polymer. Thus, suitably the knitted body and suitably the scaffold consists essentially of, and preferably consists of, a biocompatible polymer yarn.

PCL(the biocompatible polymer) may be present as a homopolymer or a copolymer. PCL (the biocompatible polymer), may be present as part of a blend.

Alternatively, or additionally, PCL (the biocompatible polymer) may comprise one or more of - e.g. as a blend or copolymer - poly(lactic acid) (PLA) [in any one or more of its isomer forms: PLLA, PDLA and PDLLA], poly(glycolic acid) (PGA), poly(lactide-coglycolide) (PLGA) [wherein the lactide component can be any one or more of the PLA isomers PLLA, PDLA and PDLLA] or poly(hydroxybutyrate) (PHB).

Nevertheless, it is preferred that the biocompatible polymer consists essentially of and preferably consists of PCL. Preferably the PCL is a homopolymer. Thus, suitably the yarn, suitably the knitted body and suitably the scaffold consists essentially of, and preferably consists of, PCL. Thus, suitably the knitted body and suitably the scaffold consists of a PCL yarn.

Preferably the polymer, suitably PCL, has a MW (Mn) of at least 10,000, more preferably at least 30,000 and most preferably at least 60,000. Preferably the MW (Mn) is no more than 200,000, more preferably no more than about 100,000. A particularly preferred MW (Mn) range is 60,000 to 100,000. A MW (Mn) of about 80,000 is especially preferred.

In the embodiments according to the claims, the second length is at least 10% greater than the first length. In other words, in these embodiments, the apertures are open at and between a configuration in which the knitted body is unloaded and a configuration in which the knitted body is loaded; wherein when unloaded the knitted body is of a first length, and when loaded the knitted body is of a second length which is at least 10% greater than the first length.

In some embodiments, the second length is at least 12% greater than the first length. In some embodiments, the second length is at least 15% greater than the first length. In some embodiments, the second length is at least 20% greater than the first length.

In some embodiments, the yarn comprises a plurality of fibres comprising the biocompatible polymer. Suitably, the fibres consist essentially of and preferably consist of the biocompatible polymer. Thus, suitably the yarn, suitably the knitted body and suitably the scaffold consists essentially of, and preferably consists of, biocompatible polymer fibres.

In some embodiments, the yarn comprises a plurality of fibres comprising PCL. Suitably, the fibres consist essentially of and preferably consist of PCL. Thus, suitably the yarn, suitably the knitted body and suitably the scaffold consists essentially of, and preferably consists of, PCL fibres.

In preferred embodiments, the fibres are made by electrospinning. This provides a way of generating fibres of a controlled diameter, including long, continuous fibres of a controlled diameter. A bundle of electrospun fibres is particularly more robust than monofilament yarn and can provide additional stretch.

Preferably the plurality of fibres are aligned. In other words, it is preferred that the fibres making up the yarn are aligned, i.e. do not have a random orientation. Suitably at least 50% of the fibres that make up the yarn are aligned. More preferably at least 75% of the fibres that make up the yarn are aligned; and most preferably at least 90% are aligned.

Suitably the plurality of fibres (for example, at least 50% of the fibres that make up the yarn) are aligned such that their longitudinal axes lie within 30°, preferably within 20°, more preferably within 10°, and most preferably within 5° of each other.

Suitably the plurality of fibres (for example, at least 50% of the fibres that make up the yarn) are substantially parallel. Suitably at least 50% of the fibres that make up the yarn are substantially parallel. More preferably at least 75% of the fibres that make up the yarn are substantially parallel; and most preferably at least 90% are substantially parallel.

Suitably the yarn, suitably the knitted body, and suitably the scaffold consists essentially of and preferably consists of the plurality of fibres.

In preferred embodiments, the yarn is formed by twisting the plurality of fibres. The provision of a twist facilitates the growth of tendon cells, especially tenocytes, along the yarn. Suitably the yarn is such that the fibres form a helix. Preferably the helix angle (the angle formed between the direction of the fibres and the longitudinal axis of the fibre bundle) is in the range of 10° to 80°, more preferably 20° to 80°, and most preferably 20° to 60°. Additionally or alternatively, it is preferred that the amount or extent of twisting is selected so as to provide at least 100 turns per metre, more preferably at least 250 turns per metre, more preferably at least 400 turns per metre, more preferably at least 700 turns per metre, more preferably at least 900 turns per metre, and most preferably at least 1000 turns per metre. Embodiments having such an extent of twist provide improved mechanical properties, for example with reference to one or more of modulus, tensile strength and strain. Embodiments demonstrate good elongation performance whilst benefiting from improved mechanical properties (for example, they may have improved tensile strength, but they remain capable of significant extension, thereby mimicking natural tendon tissue). Twists can be in either the "S" or "Z" direction.

The average diameter of the fibres of the yarn can be used to control not only the biomechanical properties of the scaffold but preferably also the effectiveness of the scaffold as an environment for encouraging cell growth. Fibre diameters in the nano scale (i.e. <1 µm) are particularly effective, as such fibres provide biomimicry with (for example) collagen fibrils for cells. Preferably the average diameter of the fibres is less than 1 µm. More preferably the average diameter of the fibres is in a range of 400 nm to 850 nm. Even more preferably the average diameter of the fibres is in a range of 550 nm to 850 nm. Most preferably the average diameter of the fibres is approximately 700 nm.

An advantage of the present invention is that properties of the scaffold, including the biomechanical properties and suitably the effectiveness of the scaffold in encouraging cell growth, can be controlled by adjusting the average diameter of the yarn. It is desirable for the yarn to be as fine as possible to prevent a tendon from bulking out, and to maximise aperture area. However, if the yarn is excessively fine, it is difficult to knit the yarn without it snapping, and the knitted body is prone to collapsing. Preferably the average diameter of the yarn is less than 500 µm. More preferably the average diameter of the yarn is less than 300 µm. Most preferably the average diameter of the yarn is in a range of 225 µm to 275 µm.

A knitted body may be selected of an appropriate length based on the tendon that is to be repaired. The length is defined as the distance from a first end to a second end of the knitted body, which is the greatest of the dimensions of the knitted body. It is desirable for the length of the knitted body to be a high as possible, whilst allowing for strong matrix anchoring either side of a cut in a tendon. In preferred embodiments, the first length of the knitted body (i.e. the length when the knitted body is unloaded) is in a range of 15 mm to 25 mm. In more preferred embodiments, the first length of the knitted body is in a range of 18 mm to 22 mm. In most preferred embodiments, the first length of the knitted body is approximately 20 mm.

A knitted body may be selected of an appropriate width based on the tendon that is to be repaired. The width is defined as the distance from a first edge to a second edge across the cross-section of the three-dimensional knitted body. In preferred embodiments, the width of the knitted body when unloaded is in a range of 1.5 mm and 2.5 mm. A knitted body of width greater than 2.5 mm can result in the tending bulking out, which can lead to limited motion and poorer repair. In more preferred embodiments, the width of the knitted body when unloaded is in a range of 1.9 mm to 2.1 mm. In most preferred embodiments, the width of the knitted body when unloaded is approximately 2.0 mm.

A knitted body may be selected having an appropriate number of knitted rows based on the tendon that is to be repaired. In some embodiments, the number of rows is in a range of 4 to 28. In preferred embodiments, the number of rows is in a range of 8 to 24. In more preferred embodiments, the number of rows is in a range of 12 to 20. In most preferred embodiments, the number of rows is approximately 16. A knitted body having 16 rows is particularly preferable for hand tendon repair.

Suitably the apertures of the knitted body are arranged in a repeating structure. Preferably the apertures are arranged as a regular or ordered network or array. The present inventors have found that a regular repeating structure may assist in promoting cell growth along and within the scaffold. Such a structure also provides desirable biomechanical properties.

Preferably, the apertures individually have an area of at least 10,000 µm² in the first configuration and an area of at least 10,000 µm² in the second configuration. More preferably, the apertures individually have an area of at least 11,000 µm² in the first configuration and an area of at least 11,000 µm² in the second configuration. Most preferably, the apertures individually have an area of at least 12,000 µm² in the first configuration and an area of at least 12,000 µm² in the second configuration. In some embodiments, the apertures individually have an area of at least 20,000 µm² in the first configuration and an area of at least 20,000 µm² in the second configuration.

In some embodiments, all apertures of the knitted body fulfil at least one of these limitations relating to area. In some embodiments, some apertures of the knitted body fulfil at least one of these limitations relating to area and other apertures of the knitted body do not fulfil at least one of these limitations.

In some embodiments, at least 20% of the apertures individually have an area of at least 10,000 µm² in the first configuration. In preferred embodiments, at least 35% of the apertures individually have an area of at least 10,000 µm² in the first configuration. In more preferred embodiments, at least 50% of the apertures individually have an area of at least 10,000 µm² in the first configuration. In most preferred embodiments, at least 65% of the apertures individually have an area of at least 10,000 µm² in the first configuration.

In some embodiments, at least 10% of the apertures individually have an area of at least 20,000 µm² in the first configuration. In preferred embodiments, at least 20% of the apertures individually have an area of at least 20,000 µm² in the first configuration. In more preferred embodiments, at least 30% of the apertures individually have an area of at least 20,000 µm² in the first configuration. In most preferred embodiments, at least 40% of the apertures individually have an area of at least 20,000 µm² in the first configuration.

In some embodiments, at least 5% of the apertures individually have an area of at least 30,000 µm² in the first configuration. In preferred embodiments, at least 10% of the apertures individually have an area of at least 30,000 µm² in the first configuration. In more preferred embodiments, at least 15% of the apertures individually have an area of at least 30,000 µm² in the first configuration. In most preferred embodiments, at least 20% of the apertures individually have an area of at least 30,000 µm² in the first configuration.

In some embodiments, at least 10% of the apertures individually have an area of at least 10,000 µm² in the second configuration. In preferred embodiments, at least 20% of the apertures individually have an area of at least 10,000 µm² in the second configuration. In more preferred embodiments, at least 30% of the apertures individually have an area of at least 10,000 µm² in the second configuration. In most preferred embodiments, at least 40% of the apertures individually have an area of at least 10,000 µm² in the second configuration.

In some embodiments, at least 5% of the apertures individually have an area of at least 20,000 µm² in the second configuration. In preferred embodiments, at least 8% of the apertures individually have an area of at least 20,000 µm² in the second configuration. In more preferred embodiments, at least 12% of the apertures individually have an area of at least 20,000 µm² in the second configuration. In most preferred embodiments, at least 15% of the apertures individually have an area of at least 20,000 µm² in the second configuration.

The above limitations relating to aperture area are particularly suitable for collagenous extracellular matrix ingrowth. It is significant that these apertures remain open under loading.

In preferred embodiments, the tissue repair scaffold comprises a first tendril extending from a first end of the knitted body. In some embodiments, the tissue repair scaffold also comprises a second tendril extending from a second end of the knitted body. It is preferred that the first tendril and (if present) the second tendril are made of the same yarn as the knitted body, such that the first tendril, knitted body and (if present) the second tendril comprise the same continuous piece of yarn. Although extending from the knitted body, the tendrils are not considered part of the knitted body with regard to its definition. The tendrils allow the scaffold to be passed through the interior of a tendon with a needle, and allow subsequent knotting once the scaffold is placed *in situ,* hindering movement out of position.

In preferred embodiments, the tissue repair scaffold is made from a single piece of yarn.

Preferably the length of the first tendril is in a range of 50 mm to 150 mm. More preferably the length of the first tendril is in a range of 90 mm to 110 mm. Most preferably the length of the first tendril is approximately 100 mm.

Preferably the length of the second tendril is in a range of 50 mm to 150 mm. More preferably the length of the second tendril is in a range of 90 mm to 110 mm. Most preferably the length of the second tendril is approximately 100 mm.

In some embodiments, the tissue repair scaffold comprises collagen gel. The collagen gel suitably improves the growth of tendon cells (especially tenocytes) along and/or within the scaffold as compared to the scaffold without collagen gel.

In a second aspect, the present invention provides a tissue repair device comprising a surgical cord attached to the tissue repair scaffold of the first aspect. The surgical cord provides a means of holding two ends of a cut or severed tissue together, and delivering the scaffold.

Suitably the surgical cord is a suture. In preferred embodiments, the suture is a monofilament suture. In some embodiments, the suture is barbed. In some embodiments, the suture comprises the same biocompatible polymer as the knitted body. In some embodiments, the suture comprises PCL.

In preferred embodiments, the surgical cord is attached to the tissue repair scaffold of the first aspect, such that the surgical cord extends approximately parallel to the length direction (tendon direction) of the knitted body, which can facilitate delivery of the scaffold. Preferably, the surgical cord is attached to the tissue repair scaffold such that the surgical cord is alongside the knitted body. Alternatively, the surgical cord may be attached to the tissue repair scaffold such that the surgical cord passes through the knitted body.

In preferred embodiments, the surgical cord is attached to the tissue repair scaffold by a knot. Preferably the knot is formed from yarn at an end of the knitted body, such that (if present) a tendril extends from the knot. In more preferred embodiments, the surgical cord is attached to the tissue repair scaffold by two knots. It is preferred that the two knots are formed from yarn at respective ends of the knitted body.

Alternatively, the surgical cord may be attached to the tissue repair scaffold by solvent sealing. Alternatively, the surgical cord may be attached to the tissue repair scaffold by heat sealing. In some embodiments, the surgical cord is attached to the tissue repair scaffold by a combination of two or more of a knot, solvent sealing and heat sealing.

In a third aspect, the present invention provides the tissue repair scaffold of the first aspect for use in a method of treatment of the human or animal body. Suitably the method is a method of treatment by surgery. Suitably the method comprises treating a damaged tendon.

Preferably the method comprises the step of attaching, preferably grafting, the tissue repair scaffold to a damaged tendon. Typically, this might be achieved by suturing the tissue repair scaffold to a damaged tendon.

In the case of treatment of animals, the animal is preferably a horse (e.g. a racehorse). Domestic pets such as one or more of dogs, cats and horses are preferred subjects for treatment. Animals in captivity, for example zoo animals, are also preferred subjects for treatment.

In a fourth aspect, the present invention provides a method of making a tendon repair scaffold as defined in claim 13.

In the embodiments according to the claims, the yarn is knitted using a weft knitting process.

In preferred embodiments, the yarn is knitted using a three-needle process.

Suitably a three-needle weft knitting process is used.

Preferably the yarn is knitted using a flatbed knitting machine. Preferably the yarn is knitted using a 10 gauge knitting machine. Suitably the yarn is knitted using a 10 gauge flatbed knitting machine.

In some embodiments, the yarn is knitted such that a first tendril extends from a first end of the knitted body. In some embodiments, the yarn is knitted such that a second tendril extends from a second end of the knitted body.

In some embodiments, the tissue repair scaffold of any of the aspects is provided in a sterile enclosure, for example a sterile packet. Suitably the enclosure is hermetically sealed.

Thus in a fifth aspect, the present invention provides a tissue repair scaffold according to any one of the aspects herein, wherein the tissue repair scaffold is provided in a sterile enclosure.

It is envisaged that the tissue repair scaffold may be provided in a variety of different sizes and morphologies, for example in a number of "off-the-shelf" configurations, so as to enable a surgeon to select the most appropriate scaffold for the tissue to be repaired. Thus in a sixth aspect, the present invention provides a kit comprising a plurality of tissue repair scaffolds, each scaffold being a scaffold according to any one of the aspects herein, wherein each scaffold is provided in a sterile enclosure. Suitably at least some of the scaffolds are different, e.g. have different dimensions and/or morphologies.

In a seventh aspect, the present invention provides a tissue repair scaffold made according to the method of the fourth aspect.

The invention includes the combination of the aspects and preferred features described except where such a combination is clearly impermissible or expressly avoided. Furthermore, any of the optional or preferred features of any one of the aspects may apply to any of the other aspects. In particular, optional features associated with a method or use may apply to a scaffold or device, and vice versa.

### Summary of the Figures

Embodiments and experiments illustrating the principles of the invention will now be discussed with reference to the accompanying figures in which:
**Figure 1** shows a typical currently clinically used technique of a Kessler suture knot to repair two cut tendon ends.
**Figure 2** shows an SEM micrograph of a known yarn formed by plaiting a plurality of primary fibre bundles.
**Figure 3** shows a schematic of the electrospinning apparatus used to make fibres, and the rotating mandrel used to align the fibres for inclusion in scaffolds of the present invention.
**Figure 4** shows an SEM micrograph of electrospun aligned fibres formed by a method of the present invention.
**Figure 5** shows an SEM micrograph of a yarn formed by twisting electrospun aligned fibres.
**Figure 6** shows a three-needle arrangement on a conventional knitting machine.
**Figure 7** shows an SEM micrograph of a knitted body formed by knitting the yarn of twisted fibres.
**Figure 8** shows an image of a tissue repair scaffold of the present invention.
**Figure 9** shows the position of the tissue repair scaffold to augment a current clinically used suture knot.
**Figure 10** shows the position of the tissue repair scaffold when used with a micro-barbed surgical cord.
**Figure 11** shows an image of a tissue repair device of the invention inserted into a human cadaver hand tendon.
**Figure 12** shows a histogram of the number of apertures across different aperture areas for a comparative plaited structure.
**Figure 13** shows a histogram for the number of apertures across different aperture areas for a knitted structure according to the present invention.
**Figure 14** shows a bar chart of the coverage of type-I collagen and fibronectin for loaded plaited and knitted structures after 21 days in an *in vitro* culture.
**Figure 15** shows a bar chart of the maximum load for cell-seeded plaited and knitted structures after 28 days in an *in vitro* culture.
**Figure 16** shows the variance in number average molecular weight (Mn) and weight average molecular weight (Mw) over the course of a 12-week rabbit study.
**Figure 17** shows the percentage reduction of surface area compared with a control at different portions, using ultrasound in a 12-week sheep study.
**Figure 18** shows a comparison of collagen type-I results over 12 months from histological analysis of sheep tendon.
**Figure 19** shows a comparison of decorin results over 12 months from histological analysis of sheep tendon.
**Figure 20** shows a comparison of inflammatory cell results over 12 months from histological analysis of sheep tendon.

### Detailed Description of the Invention

Aspects and embodiments of the present invention will now be discussed with reference to the accompanying figures.

Examples of tendon repair scaffolds are described herein, comprising a three-dimensional knitted body made of yarn formed by twisting a plurality of aligned electrospun PCL fibres. These fibrous constructs are intended to mimic both the morphological anatomy and the biomechanical properties of natural human tendon. This tissue is known to be composed of a hierarchical organisation of aligned collagen fibres. In embodiments, whilst the fibres contained within the PCL yarn are not of the same size as the collagen fibres, the dimensions and morphology of the yarn closely resembles those within the natural tendon tissue.

The scaffolds described herein are biodegradable and/or bioresorbable. This suitably eliminates the need for secondary surgery. Furthermore, in embodiments, the rate of degradation matches the rate of new tissue formation. Preliminary studies suggest that the degradation rates are suitable for accommodating natural healing times for tendons of about three months.

Testing of embodiments has shown that the scaffolds are able to withstand high tensile loads and demonstrate flexibility. This latter characteristic is particularly promising given that some tendons are required to bend round bony prominences.

Described herein are studies which demonstrate how electrospinning of fibres can be used to produce the scaffolds of the present invention. Variation of the parameters associated with electrospinning can be used to control the fibre characteristics.

The electrospun fibres described herein are drawn off a mandrel and twisted onto a bobbin. This is then used to form the twisted multifilament yarn. The yarn is subsequently knitted into a structure with a predetermined pattern, by a weft knitting process using three needles, giving rise to a slim cross-section which is broadly circular. If other knitting styles are used, then a different cross-section is generated. For example, the use of four needles gives rise to a flatter, more ribbon-like cross-section. Moreover, a four-needle process generally results in greater deformation of yarn during knitting.

### (1) Fibre formation

### (a) Electrospinning and selection of parameters

Electrospinning provides a way of generating fibres of controlled diameter. In particular, electrospinning enables the fabrication of long, continuous fibres of controlled diameter. At its simplest, the application of a high voltage to a polymer solution within a syringe causes expulsion of a polymer jet towards an earthed collector.

It is possible to electrospin a wide range of polymers, including biocompatible polymers.

A number of parameters can be used to control the properties of the fibres formed using electrospinning. The following parameters are mentioned as particularly useful in controlling the collected fibres:
1. Solvent
2. Molecular weight of polymer
3. Concentration of polymer-solvent solution
4. Applied voltage
5. Tip to collector distance
6. Flow rate of polymer-solvent solution

These and other parameters are discussed in more detail below.

### Solvent

Suitable solvents include acetone, chloroform, dichloromethane (DCM), 1,1,1,3,3,3-hexafluoroisopropanol (HFIP) and tetrahydrofuran (THF).

Solvents with a higher dielectric constant yield a greater number of fibres, due to the charge repulsion interactions which occur as the polymer jet travels towards the collector.

### Molecular weight of polymer

A suitable molecular weight (Mn) is in the range 10,000 to 100,000, but other molecular weights can be used, as described herein.

Molecular weight can have a significant effect on the fibre morphology. PCL of higher molecular weight (Mn 80,000) is found to minimise the occurrence of "beads" along the fibres.

### Concentration of polymer-solvent solution

Suitable concentrations are in the range 3% w/v to 15% w/v, preferably 5% w/v to 10% w/v.

Fibre diameter can be tailored by altering the solution concentration. Generally, it is found that higher concentrations result in fibres of greater diameter.

### Voltage

The voltage applied to the polymeric solution has a direct effect on fibre morphology. High voltage causes the polymer jet to be emitted with rapid acceleration, limiting the jets flight time and subsequently decreases the amount of stretching and solvent evaporation prior to collector impact [Ramakrishna *et al.,* 2005]. The resulting fibres may be thicker and contain high levels of residual solvent. The flight time of the polymer jet is, however, dependent upon the tip to collector distance.

Keeping the applied voltage comparatively low (e.g. 15kV) is effective in producing electrospun fibres of fine, submicron diameter. Nevertheless, it is desirable to avoid much lower voltages so as to ensure sufficient electro-static charging of the polymer solution and ejection of a polymer jet.

### Needle-tip to collector distance

Varying the distance between the needle-tip to the collector has an important role in determining fibre characteristics. A short deposition distance reduces the polymer jet flight time, limiting the rate of solvent evaporation and polymer stretching; often resulting in the fabrication of thick, merged fibres. Generally, a minimum distance, which allows significant drying and stretching of the jet, is required for the production of long, fine fibres [Reneker *et al*., 2000].

The deposition distance between the needle-tip and collector should be long enough to ensure adequate time for the polymer jet to undergo sufficient stretching and solvent evaporation prior to its impact. Suitable distances are in the range of 10 cm to 20 cm, preferably 12 cm to 15 cm.

### Flow rate of polymer-solvent solution

The applied flow rate determines the quantity of polymeric solution available to the electrospinning process. Generally, high flow rates yield fibres of larger diameter. This is because the greater volume of solution pumped out may not have sufficient time for solvent evaporation and adequate stretching of the jet prior to contact with the collector.

It is desirable to use a low flow rate, as high flow rates may result in fibres of wider diameter (e.g. larger than 1 µm).

### Solution viscosity

The viscosity of solution is directly affected by the concentration of polymer present. If the polymer concentration is high, greater quantities of polymer chains are present, increasing the number of chain entanglements with solvent molecules and ultimately raising solution viscosity. A polymer's molecular weight also affects solution viscosity. A polymer of low molecular weight reduces the number of solvent/polymer entanglements because of the shorter length chains, and hence decreases solution viscosity. Fibre production is dependent on the concentration of solution being electrospun.

Generally, if the concentration is too low bead formation as opposed to fibre production is observed; and if too high, pumping of the solution will be difficult and fabricated fibres are mostly micrometre in diameter.

### Solution conductivity

The solvent chosen to dissolve the polymer has a significant role in the level of conductivity present within the solution, and this directly affects the fibre morphology generated from the electrospinning process. Solvents with high dielectric constants cause the emitted polymer jet to experience increased longitudinal force brought about by the higher accumulation of charge present within the polymeric solution [Wannatong *et al.,* 2004]. Consequently the polymer jet experiences a greater degree of charge repulsion, leading to an increased level of stretching and elongation, resulting in fibres of finer diameter [Fong *et al.,* 1999].

### Surface tension

The surface tension of the polymeric solution must be overcome in order for the electrospinning process to be initiated. The polymeric solutions viscosity directly affects its surface tension; high viscosity reduces the surface tension due to significant entanglement between solvent molecules and polymer chains preventing molecule clustering [Shawon *et al.,* 2004].

### Humidity

The humidity surrounding the electrospinning process can have a significant effect on fibre morphology, in terms of surface porosity: rising levels of humidity lead to an increase in pore size, number and distribution over the fibre surface [Casper *et al.,* 2004].

### Temperature

Increasing the polymeric solution temperature may result in a faster rate of fibre deposition and fibres may therefore have a decreased diameter. These effects may be attributed to the reduced viscosity of polymeric solution caused by decreased entanglements between polymer and solvent molecules as a result of polymer chain expansion [Mit-uppatham *et al.,* 2004].

### Specific parameters

Unless otherwise stated, the electrospinning parameters employed for the fabrication of all electrospun fibre matrices described herein were as follows: voltage 15 kV (Series 120 Watt regulated high-voltage DC power supply, Glassman High Voltage, Inc.), flow rate 4.5 mL/hour (SP230IW2, World Precision Instruments), needle tip (ø 0.8 mm, BD Microlance) to collector distance 15 cm, solution concentration 8% w/v polycaprolactone (PCL, Purasorb PC 12) (80,000 Mn) [Purac Corbion] in HFIP [Sigma Aldrich], humidity 50%, temperature 23 °C.

### (b) Fibre orientation

### Fibre alignment and collector method

The orientation of fibres deposited from the electrospinning process is dependent upon their method of collection. Fibre alignment is determined by the angle between the fibre and the direction of alignment - the smaller the angle, the greater the alignment.

Random, non-woven arrangements of fibres are created when the collector is an earthed stationary plate.

Purposefully orientated fibres can be fabricated by electrospinning between the gap of two fixed metal plates or onto a mandrel rotating at an optimised speed. The method described herein uses a rotating mandrel. Generally, the mandrel must be rotated at sufficient speed so as to ensure that rotation speed is not too slow compared to the speed of fibre emission, in which case alignment may be inhibited. Similarly, if rotation is too fast, fibre breakage can occur [Huang *et al.,* 2003].

Alternatively, collection of fibres between two parallel earthed plates, also known as "gap method of alignment" may be used to produce aligned fibres [Dzenis, 2004].

Fibrous yarns containing aligned individual fibres that are grouped together can also be fabricated by spinning the polymer solution directly into an earthed liquid reservoir [Smit *et al.,* 2005]. The network of fibres collected on the liquid surface is drawn off and into the air. Fibres align and coalesce due to the effects of surface tension between the fibres and liquid during the drawing process. Three-dimensional fibrous bundles are the end product after lifting the fibres off the liquid surface.

The various methods for fibre collection resulted in different fibre orientation. The stationary plate produces fibres with least alignment. Collection on the rotating mandrel results in fibres of greatest alignment.

### Optimising rotation speed for creating aligned fibres on a mandrel

The orientation of fibres when collected on a mandrel depends on the mandrel's rotation speed. For a narrow mandrel (width = 30 mm; diameter = 120 mm), low speeds of 300 RPM result in fibre deposition similar to the fibrous networks collected on stationary plates. If the speed of rotation is too fast, the fibres are predominantly random in appearance. At an optimal speed, the fibres are collected parallel to the direction of rotation. 500-600 RPM (e.g. 500 RPM) results in the greatest alignment.

For a wide mandrel (width = 90 mm; diameter = 65 mm), the optimum rotation speed for the alignment of fibres is different from that of a narrow mandrel. For the wide mandrel, speeds of 1200 RPM and greater are employed to generate sufficiently aligned fibres. Alignment may be further increased at rotation speeds above 1800 RPM, giving a lower angle of alignment and hence greater degree of parallelism to the longitudinal axis.

### (c) Surface characteristics

The wettability of the scaffold surface is a characteristic of the scaffold that can be adjusted to suit the function of the scaffold. Suitably, to encourage cell attachment, the exterior of the material is hydrophilic or wettable as this will permit cells to contact with the surface over a greater area to allow attachment and spreading, and for providing cells with an environment similar to their natural environment. To discourage cell attachment or protein adhesion, however, hydrophobic or non-wetting surfaces can be created. The skilled reader is familiar with appropriate surface treatments.

### (2) Scaffold formation

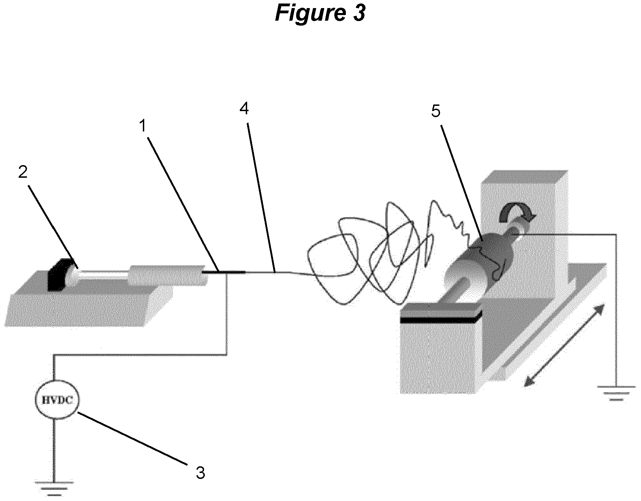

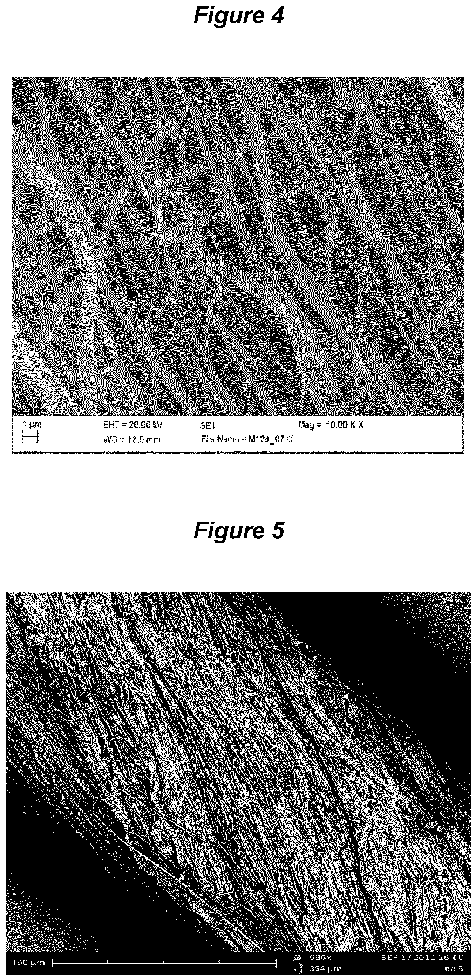

Figure 3 shows a schematic of the electrospinning apparatus used to make PCL fibres, and the rotating mandrel used to align the PCL fibres for inclusion in scaffolds of the present invention. The electrospinning apparatus comprises a syringe needle (1) attached to a syringe pump (2), powered by a high-voltage DC supply (3). The mandrel (5), which has a rotating and translating element, collects aligned PCL fibres (4). An SEM micrograph of electrospun aligned PCL fibres formed by this method is shown in Figure 4.

Yarn is then created in an automated machine process by pulling the aligned PCL fibres from the mandrel and creating a twist. The resultant yarn is collected on a bobbin, typically in a length of greater than 1 m. An SEM micrograph of yarn formed by this method is shown in Figure 5.

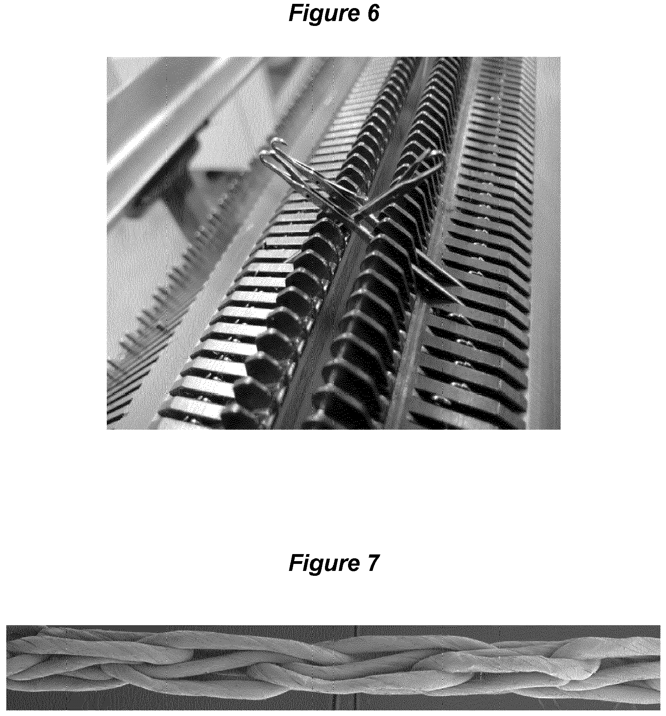

Subsequently, the yarn is knitted in a weft knitting process to produce the tissue repair scaffold, using a three-needle arrangement on a conventional 10 gauge flatbed knitting machine, as shown in Figure 6, with proprietary bobbin holder/tensioner. A bobbin containing yarn is loaded into the moving holder/tensioner. The loose end of yarn is fed through a tensioning arm and three subsequent feed holes. The loose end is passed through the knitting beds. From underneath the knitting beds, the yarn is taken and the end is clamped into a clamp attached to the knitter. The knitter is moved across the needles from right to left to cast on. The knitter is then moved across the needles from left to right to knit a first row, and from right to left to knit a second row. This is repeated until 16 rows are knitted to produce a knitted body of 20 mm in length. The two trailing lengths of yarn from either end of the knitted body are each trimmed to 100 mm in length.

The tissue repair scaffold comprises a knitted body as shown by the SEM micrograph in Figure 7. The scaffold has a broadly circular cross-section, and comprises a number of apertures in between different segments of the yarn resulting from the knitted structure. The scaffold also has a number of significantly smaller, micropores generated by twists in a segment of yarn.

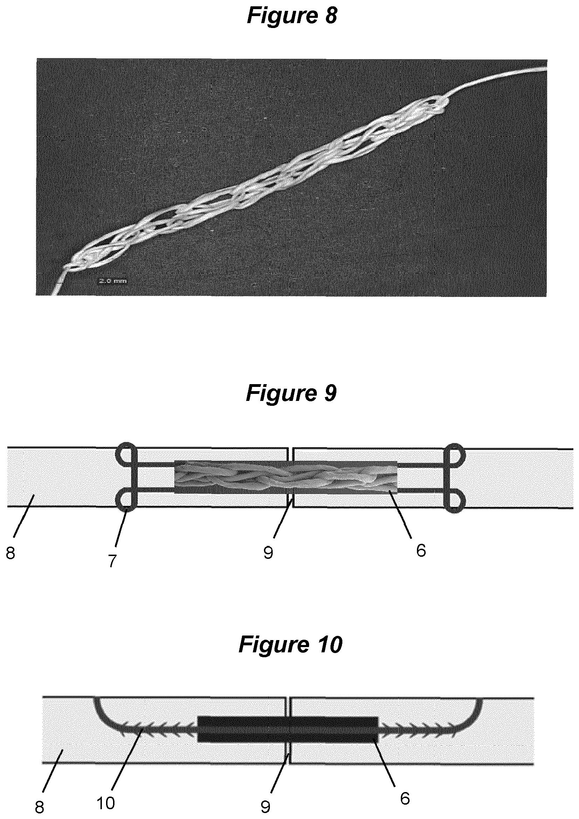

An image of a tissue repair scaffold is shown in Figure 8, in which the scale bar represents 1 mm. The scaffold is made from a single piece of yarn. The scaffold comprises three distinct regions: a trailing length of electrospun PCL yarn (first tendril), the knitted body, followed by a second tendril.

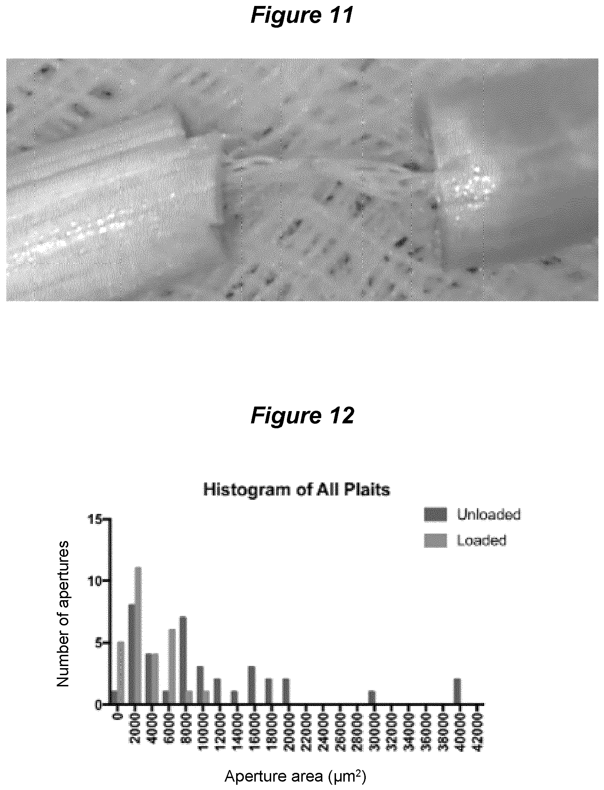

As exhibited by the schematic in Figure 9, the tissue repair scaffold (6) can be used to augment a current clinically used suture knot (7). The scaffold is inserted in the centre of a tendon (8), across a cut (9) in the tendon. The scaffold is attached to the suture knot such that the scaffold is alongside and approximately parallel to the suture. An alternative, using a micro-barbed surgical cord (10) in place of the suture knot, is shown in the schematic in Figure 10. The scaffold together with the suture knot or micro-barbed surgical cord can be considered as a tissue repair device. An image of a device inserted into a human cadaver hand tendon is shown in Figure 11 (note that the gauze-like structure in the background of the image is not part of the device).

### (3) Exemplified scaffolds

The specifications of exemplified tissue repair scaffolds formed by the method described above are shown in Table 1:

**Table 1**

| **Scaffold specifications** | |
|---|---|
| PCL fibre diameter (nm) | 700 (± 20%) |
| Yarn diameter (µm) | 225-275 |
| Tendril lengths (mm) | 100 |
| Knitted body length (mm) | 20 |
| Knitted body width (mm) | 2 |

One knitted body of a scaffold of these specifications was scanned with µCT. The scan was analysed using a semi-automated Avizo (voxel number based) procedure, which was repeated three times. The scan was divided into 5 different regions to calculate the final porosity, comprising microporosity and macroporosity.

Microporosity is the result of the void generated by twists within the yarn, divided by the solid material. Macroporosity is the void space percentage against the whole volume of the knitted body, imagined as if it were wrapped in an envelope (so is essentially the sum of the solid and void space). Macroporosity relates to the apertures in the knitted structure as described herein. The procedure does not account for the space between individual electrospun fibres, as the resolution of the machine does not distinguish between fibres.

It was found that measured macroporosity of the knitted body was 53.65%. The measured microporosity was 3.99%.

Three scaffolds of the above specifications were taken and placed under a microscope, and the aperture area of each knitted body (unloaded) was measured at 4× magnification using ImageJ (free-hand selection). The knitted bodies were then manually pulled to a length 10% greater than the length when unloaded, and the aperture area of each knitted body (loaded) was then measured again in the same way.

For comparison, three scaffolds having a structure of plaited yarn comprising PCL fibres were also taken (see Figure 10 of WO 2010/067086 and the methods disclosed therein). These comparative scaffolds were placed under a microscope, and the aperture area of each plaited structure (unloaded) was measured at 4× magnification using ImageJ (free-hand selection). The plaited structure was then manually pulled to a length 10% greater than the length when unloaded, and the aperture area of each plaited structure (loaded) was then measured again in the same way.

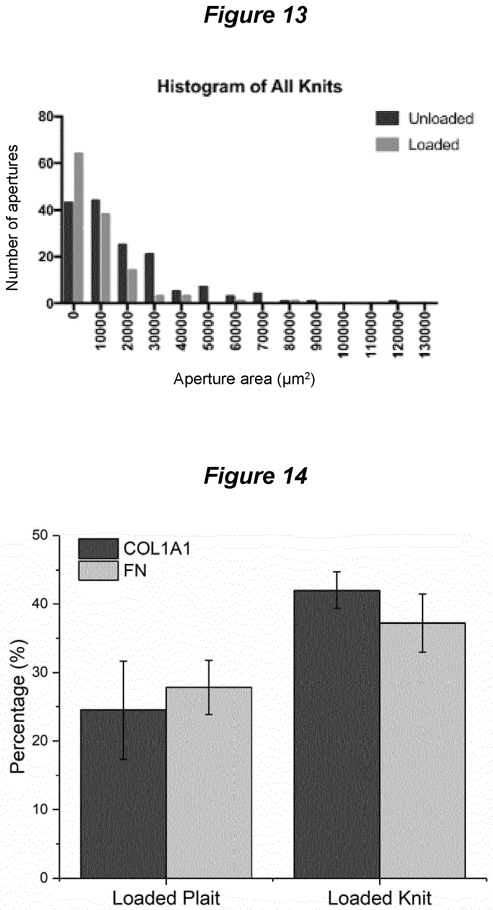

The results are exhibited in the histograms of Figures 12 and 13.

Figure 12 shows a histogram of the number of apertures across different aperture areas for all plaited structures, comparing the number of apertures when unloaded and loaded as described above. Figure 13 shows a histogram of the number of apertures across different aperture areas for all knitted bodies, comparing the number of apertures when unloaded and loaded as described above. It can be seen that the knitted body has a significantly greater number of apertures, both when unloaded and loaded. In particular, when loaded the knitted body retains almost 40 open apertures having an area in the range of 10,000-20,000 µm², and approximately 20 open apertures having an area greater than 20,000 µm². By comparison, the plaited structure has a negligible number of open apertures having an area greater than 10,000 µm² when loaded. Specifically, in this range the plaited structure has only 1 open aperture when loaded, which has an area between 10,000-12,000 µm², and has no open apertures having an area greater than 12,000 µm².

It can be concluded from these data that the knitted body possesses a greater aperture area than the plaited structure. Whereas the plaited structure is more two-dimensional and predominantly has apertures which are discrete from each other, the knitted body is a truer three-dimensional structure with interlocking pores. Although the aperture area does reduce upon loading for both structures, this is significantly more evident for the plaited structure. The knitted body retains an open-aperture structure on loading, which is more conducive for repeated loading of tissue without scissoring.

### (4) Protein deposition studies

Type-I collagen and fibronectin represent two important proteins to assess the success of tendon repairs.

Type-I collagen is the most abundant protein in tendon extracellular matrix (ECM), as it constitutes between 80% and 90% of total tendon dry mass and represents approximately 60% of the total collagen amount. Type-I collagen performs several functions, but its main role is to maintain tissue hierarchical structure, and to absorb and transmit the forces generated by muscles, preventing tissue mechanical failure. In particular, the hierarchical organisation of collagen in normal tendon plays a key role in tissue biomechanics, since it ensures that the tissue is able to withstand high tensile forces, and allows minor damage to be confined at the site rather than spreading to the entire tissue.

Fibronectin is another important protein found in tissue ECM, as it provides binding sites for cells, facilitating cellular adhesion to the matrix. This cell-protein interaction plays a key role in translating mechanical stimuli into cell responses, as fibronectin helps cells to organise collagen fibrils into bundles and to maintain the tissue hierarchical structure.

For these reasons, the success of tendon repair strategies can be evaluated by observing cell deposition of type-I collagen and fibronectin. Indeed, repaired tendons with high levels of these proteins more closely resemble the ECM structure of uninjured and healthy tendons, thus indicating a stronger and more functional repair.

Human mesenchymal stem cells (1 × 10⁶) were seeded onto each of the three knitted and three plaited PCL structures as described above, in an *in vitro* culture. The structures were each loaded so that the lengths of the structures were 10% greater than the lengths when unloaded. After 21 days in the culture, the amounts of type-I collagen (COL1A1) and fibronectin (FN) in each structure were analysed from confocal images using ImageJ and quantified. The results are provided in the bar chart in Figure 14, showing data as mean ± standard deviation. When going from the plaited structure to the knitted structure, there was an increase in coverage of collagen from 24.5% to 42.0%. Similarly, when going from the plaited structure to the knitted structure, there was an increase in coverage of fibronectin from 27.8% to 37.2%.

### (5) Tensile testing

Human mesenchymal stem cells (1 × 10⁶) were seeded onto each of six knitted and six plaited PCL structures in an *in vitro* culture. Three of each type of structure were loaded so that the lengths of the structures were 10% greater than the lengths when unloaded. The remaining three of each type of structure were not loaded.

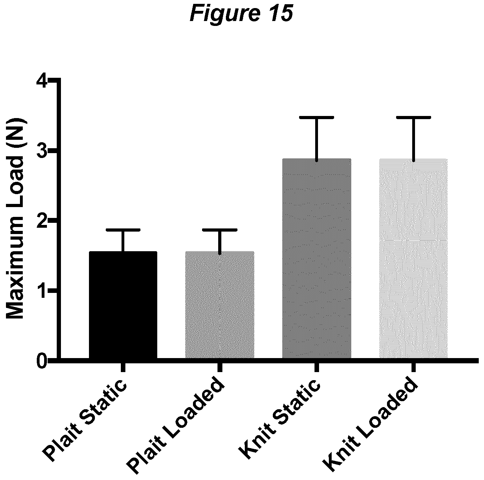

After 28 days in the culture, tensile testing was conducted with the structures, in order to determine the maximum possible load. The results are provided in the bar chart in Figure 15, showing data as mean ± standard deviation. Both the plaited and knitted structures showed little difference in maximum load in terms of whether that particular structure had been loaded or not. However, when going from the plaited structure to the knitted structure, there was an increase in maximum load from approximately 1.5 N to approximately 2.8 N.

### (6) Studies

### (a) Cytotoxic response

The cytotoxic response of the knitted scaffold was tested *in vitro* and in accordance with ISO10993-5 (Namsa, L929 cells). Epithelial cells, mesenchymal stem cells and tenocytes were cultured both in direct and indirect contact with the scaffold up to 21 days *in vitro.* All studies concluded that the scaffold is non-cytotoxic.

### (b) Murine study

PCL is biocompatible, non-cytotoxic and bioresorbs slowly. The biological safety of PCL yarns was demonstrated in a 12-month murine study. In this study, PCL yarns were delivered into the flexor digitorum longus (FDL) tendon in the hind paw of mice. Excised tissue was used as an autograft in the FDL tendon of the opposite hind paw. Positive cell infiltration, no significant increase in inflammation at any time and long-term PCL degradation were observed. Over the 12-month period, a positive presence of collagen was observed both within and around the PCL yarn.

### (c) Rabbit study

Biological safety was also demonstrated in a 12-week rabbit study, in which the local tissue response to implantation of PCL yarn was investigated relative to a standard suture material, monofilament polydioxanone (PDS). Each rabbit served as its own control, with either PCL (left hind) or PDS (right hind) implanted into the Achilles tendon.

Qualitative assessments of sections found few clear differences between the two materials in terms of the reaction elicited, including inflammation. However, a notable exception was the clear integration into PCL yarn of cells, with demonstrable progress from week 3 to 12 (whereas there was no evidence of cellular infiltration into PDS). Increased collagen and tenascin-C deposition were observed with PCL compared to PDS.

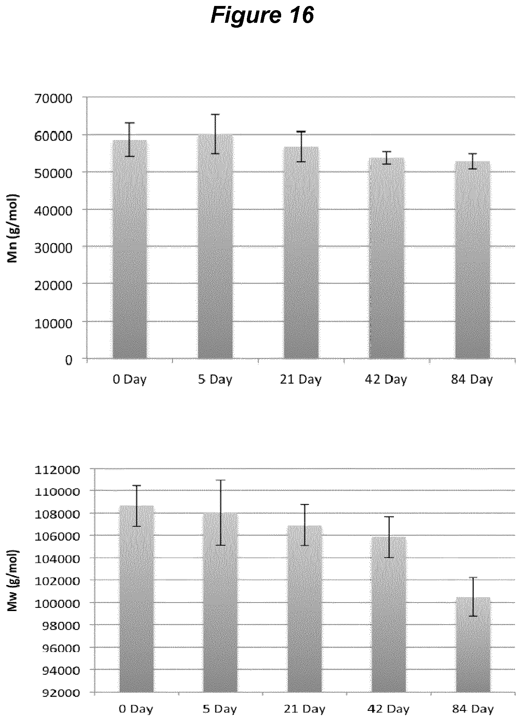

Figure 16 shows the progressive reduction in number average molecular weight (Mn) and weight average molecular weight (Mw) of PCL over the course of this study. The data are shown as mean ± standard deviation. Specifically, a 10% decrease in Mn and an 8% decrease in Mw were observed over the 12 weeks (84 days), illustrating the degradation of the PCL yarn.

### (d) Sheep efficacy studies

Studies were carried out involving two groups of ten sheep (a test group and a control group). Both groups underwent a transection of the deep digital flexor tendon of the left hind limb with a two-strand Vicryl Kessler repair with additional circumferential suture.

The test group was implanted with the novel scaffold using a curved, hollow needle. Meanwhile, the control group received an equivalent intervention but no device was implanted. The full limb was cast for 14 days.

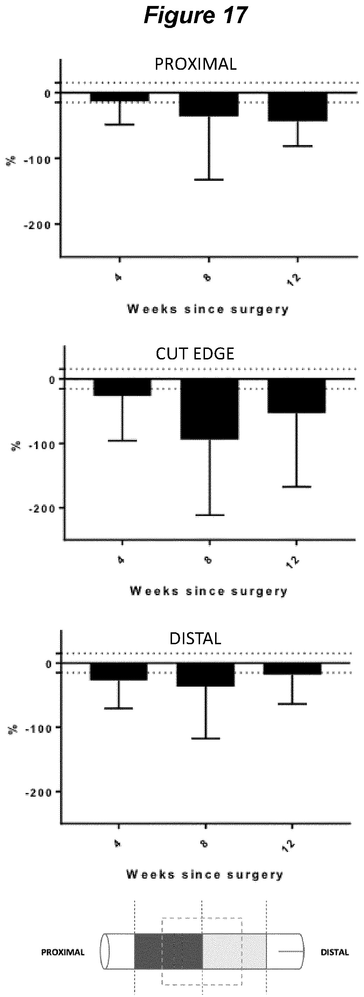

The results of a first *in vivo* study are shown in Figure 17. Using ultrasound, the percentage reduction of surface area compared with the Vicryl control was measured after 4, 8 and 12 weeks, after normalisation to the native tendon. Results were obtained for the proximal, cut edge and distal portions (the definitions of which are indicated in the figure). Good surface area reduction was observed, demonstrating an improved healing response.

A second *in vivo* study after 21 days demonstrated cell and ECM alignment across the knitted structure.

Following termination at 12 months, the tendons were harvested for mechanical testing or histological analysis. The mechanical testing showed that the repaired test tendons were strong enough to withstand a load in excess of 350 N.

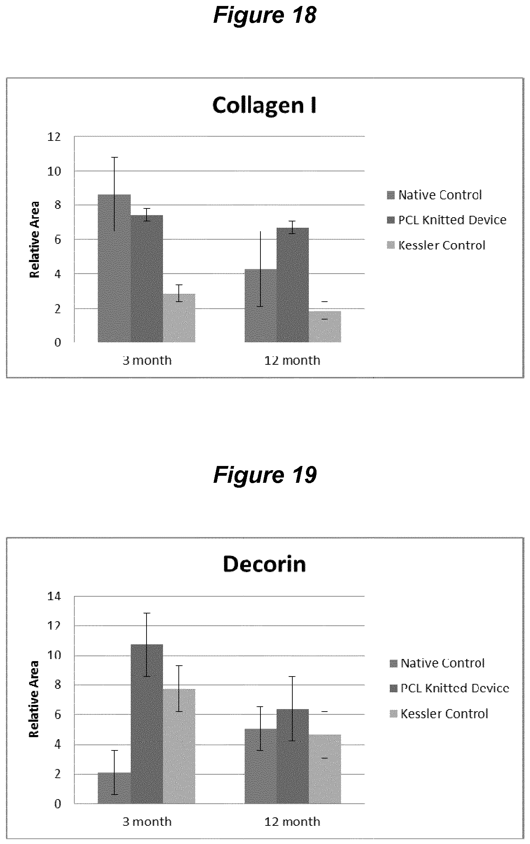

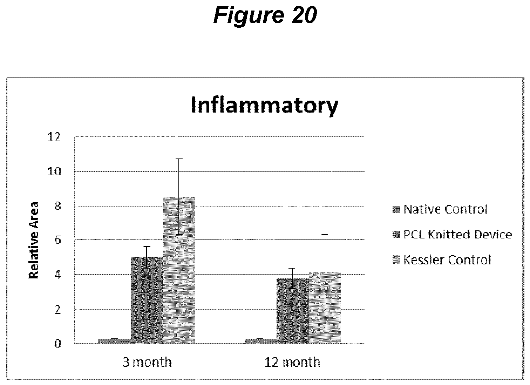

Three tendons from each group were used for histological analysis. The results are shown in Figures 18-20. It was observed by histology that ECM proteins collagen type-I and decorin (which also plays a role in matrix assembly) are significantly upregulated in the presence of the knitted PCL scaffold with suture, compared to the Kessler suture control. Specifically, there was a 160% increase at 3 months and a 260% increase at 12 months in collage type-I (see Figure 18). There was also a 39% increase at 3 months and a 38% increase at 12 months in decorin (see Figure 19). Meanwhile, there was a 41% decrease at 3 months and an 8% decrease at 12 months in inflammatory cells (see Figure 20).

The results demonstrated that the nanofibrous architecture of the PCL knitted scaffold confers contact guidance cues to the cells and ECM, and that the cells are able to penetrate through to the core of the scaffold *in vivo.*

### (7) Sterilisation and packaging

Studies have shown that there is a slight, but not significant, decrease in molecular weight of the PCL after sterilisation, accompanied by no significant change in tensile strength of the scaffold. The sterilisation was a low-temperature ethylene oxide process (Anderson Ltd, ISO 13485).

The scaffold can be packaged in Dispos-a-vent^{®} packaging (Oliver Tolas). This has two seals: one applied after device fabrication (Tyvec/foil) and one after the sterilisation process. Both seals are strong enough to meet the minimum force recommend in BS EN 868-5:2009. The test complied with international standards ISO 11607 and ASTM F88/F88M-09. A shelf-life study showed no significant difference in peel strength over 12 months.

For the avoidance of any doubt, any theoretical explanations provided herein are provided for the purposes of improving the understanding of a reader. The inventors do not wish to be bound by any of these theoretical explanations.

Any section headings used herein are for organisational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a", "an", and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about", it will be understood that the particular value forms another embodiment. The term "about" in relation to a numerical value is optional and means, for example, +/- 10%.

### References

A number of publications are cited above in order to more fully describe and disclose the invention and the state of the art to which the invention pertains. Full citations for these references are provided below.

WO 2010/067086 A2.

Cao Y L, Liu Y T, Liu W, Shan Q X, Buonocore S D, Cui L, (2002), "Bridging tendon defects using autologous tenocyte engineered tendon in a hen model", Plastic and Reconstructive Surgery, 110(5), 1280-1289.

Casper C L, Stephens J S, Tassi N G, Chase D B, Rabolt J F, (2004), "Controlling surface morphology of electrospun polystyrene fibres: effect of humidity and molecular weight in the electrospinning process", Macromolecules, 37, 573-578.

Curtis A S, Wilkinson C D, Crossan J, Broadley C, Darmani H, Johal K K, Jorgensen H, Monaghan W, (2005), "An in vivo microfabricated scaffold for tendon repair", Eur Cell Mater, 9:50-57.

Dzenis Y (2004), "Spinning continuous fibres for nanotechnology", Science, 304, 1917-1919.

Fong H, Chun I, Reneker D, (1999), "Beaded nanofibers formed during electrospinning", Polymer, 40, 4584-4592.

Huang Z M, Zhang Y Z, Kotaki M, Ramakrishna S, (2003), "A review on polymer nanofibers by electrospinning and their applications in nanocomposites", Compos Sci Technol, 63, 2223-2253.

Li W-J, Danielson K G, Alexander P G, Tuan R S, (2003), "Biological response of chondrocytes cultured in three-dimensional nanofibrous poly(ε-caprolactone) scaffolds", J Biomed Mater Res A, 67A(4), 1105-1114.

Maganaris C N, Paul J P, (1999), "In vivo human tendon mechanical properties", Journal of Physiology, 521.1, 307-313.

Magnusson S P, Hansen P, Aagaard P, Brønd J, Dyhre-Poulsen P, Bojsen-Moller J, Kjaer M, (2003), "Differential strain patterns of the human gastrocnemius aponeurosis and free tendon, in vivo", Acta Physiologica Scandinavica, 177(2), 185-195.

Mit-uppatham C, Nithitanakul M, Supaphol P, (2004), "Ultrafine electrospun polyamide-6 fibres: effect of solution conditions on morphology and average fibre diameter", Macromol Chem Phys, 205, 2327-2338.

Ramakrishna S, Fujihara K, Teo W-E, Lim T-C, Ma Z, (2005), "An introduction to Electrospinnining and Nanofibers", World Scientific Publishing Co Ltd.

Reneker D H, Yarin A L, Fong H, Koombhongse S, (2000), "Bending instability of electrically charged liquid jets of polymer solutions in electrospinning", J Appl Phys, 87(9), 4531-4547.

Shawon J, Sung C, (2004), "Electrospinning of polycarbonate nanofibres with solvent mixtures THF and DMF", J Mater Sci, 39, 4605-4613.

Smit E, Büttner U, Sanderson R D, (2005), "Continuous yarns from electrospun fibres", Polymer, 46, 2419-2423.

Smith D J (Jr), Jones C S, Hull M, Kleinert H E, (1986), "Evaluation of glutaraldehyde-treated tendon xenograft", J Hand Surg [Am], 11(1), 97-106.

Venugopal J, Ma L L, Yong T, Ramakrishna S (2005), "In vitro study of smooth muscle cells on polycaprolactone and collagen nanofibrous matrices", Cell Biology International, 29, 861-867.

Wannatong L, Sirivat A, Supaphol P, (2004), "Effects of solvents on electrospun polymeric fibres: preliminary study on polystyrene", Polymer International, 53, 1851-1859.

## Claims

1. A tendon repair scaffold comprising a knitted body, wherein
the knitted body is made of a yarn comprising polycaprolactone (PCL);
the knitted body comprises a plurality of apertures;
the apertures are open at and between a first configuration in which the knitted body is unloaded and has a first length, and a second configuration in which the knitted body is loaded and has a second length which is at least 10% greater than the first length; and
the knitted body is formed by knitting the yarn using a weft knitting process.

2. The tendon repair scaffold according to claim 1, wherein the yarn comprises a plurality of fibres comprising PCL, optionally wherein the fibres are made by electrospinning and/or wherein the plurality of fibres are aligned and/or wherein the yarn is formed by twisting the plurality of fibres.

3. The tendon repair scaffold according to claim 2, wherein the average diameter of the fibres is less than 1 µm, preferably between 400 nm and 850 nm.

4. The tendon repair scaffold according to any one of the preceding claims, wherein the average diameter of the yarn is less than 500 µm, preferably between 225 µm and 275 µm.

5. The tendon repair scaffold according to any one of the preceding claims, wherein the first length of the knitted body is between 15 mm and 25 mm.

6. The tendon repair scaffold according to any one of the preceding claims, wherein the average width of the knitted body in the first configuration is between 1.5 mm and 2.5 mm.

7. The tendon repair scaffold according to any one of the preceding claims, wherein the apertures individually have an area of at least 10,000 µm² in the first configuration and an area of at least 10,000 µm² in the second configuration.

8. The tendon repair scaffold according to any one of the preceding claims, comprising a first tendril extending from a first end of the knitted body, optionally comprising a second tendril extending from a second end of the knitted body.

9. The tendon repair scaffold according to claim 8, wherein the first tendril and, if present, the second tendril are made of the same yarn as the knitted body.

10. A tendon repair device comprising a surgical cord attached to the tendon repair scaffold according to any one of the preceding claims.

11. The tendon repair device according to claim 10, wherein surgical cord is a suture, optionally wherein the suture is barbed.

12. The tendon repair scaffold according to any one of claims 1 to 9 for use in a method of treatment of the human or animal body, wherein the method comprises treating a damaged tendon.

13. A method of making a tendon repair scaffold according to any one of claims 1 to 9, the method comprising the steps of:
(A) electrospinning a plurality of fibres comprising PCL;
(B) forming a yarn by aligning and twisting the plurality of fibres; and
(C) knitting the yarn using a weft knitting process to form a knitted body comprising a plurality of apertures.

14. The method according to claim 13, wherein the yarn is knitted using a three-needle weft knitting process.

## Patentansprüche

1. Sehnenreparaturgerüst, das einen gestrickten Körper umfasst, wobei
der gestrickte Körper aus einem Garn hergestellt ist, das Polycaprolacton (PCL) umfasst;
der gestrickte Körper eine Vielzahl von Öffnungen umfasst;
die Öffnungen bei und zwischen einer ersten Konfiguration, in der der gestrickte Körper unbelastet ist und eine erste Länge aufweist, und einer zweiten Konfiguration, in der der gestrickte Körper belastet ist und eine zweite Länge aufweist, die mindestens 10 % größer ist als die erste Länge, offen sind; und
der gestrickte Körper hergestellt wird, indem das Garn mithilfe eines Schussstrickverfahrens gestrickt wird.

2. Sehnenreparaturgerüst nach Anspruch 1, wobei das Garn eine Vielzahl von Fasern umfasst, die PCL enthalten, wobei gegebenenfalls die Fasern durch Elektrospinnen hergestellt sind und/oder wobei die Vielzahl von Fasern ausgerichtet ist und/oder wobei das Garn durch Verdrehen der Vielzahl von Fasern gebildet wird.

3. Sehnenreparaturgerüst nach Anspruch 2, wobei der durchschnittliche Durchmesser der Fasern weniger als 1 µm beträgt, vorzugsweise zwischen 400 nm und 850 nm.

4. Sehnenreparaturgerüst nach einem der vorstehenden Ansprüche, wobei der durchschnittliche Durchmesser des Garns weniger als 500 µm beträgt, vorzugsweise zwischen 225 µm und 275 µm.

5. Sehnenreparaturgerüst nach einem der vorstehenden Ansprüche, wobei die erste Länge des gestrickten Körpers zwischen 15 mm und 25 mm liegt.

6. Sehnenreparaturgerüst nach einem der vorstehenden Ansprüche, wobei die durchschnittliche Breite des gestrickten Körpers in der ersten Konfiguration zwischen 1,5 mm und 2,5 mm liegt.

7. Sehnenreparaturgerüst nach einem der vorstehenden Ansprüche, wobei die Öffnungen jeweils eine Fläche von mindestens 10.000 µm² in der ersten Konfiguration und eine Fläche von mindestens 10.000 µm² in der zweiten Konfiguration aufweisen.

8. Sehnenreparaturgerüst nach einem der vorstehenden Ansprüche, umfassend eine erste Ranke, die sich von einem ersten Ende des gestrickten Körpers erstreckt, gegebenenfalls umfassend eine zweite Ranke, die sich von einem zweiten Ende des gestrickten Körpers erstreckt.

9. Sehnenreparaturgerüst nach Anspruch 8, wobei die erste Ranke und, falls vorhanden, die zweite Ranke aus demselben Garn hergestellt sind wie der gestrickte Körper.

10. Sehnenreparaturvorrichtung, umfassend eine chirurgische Schnur, die am Sehnenreparaturgerüst nach einem der vorstehenden Ansprüche befestigt ist.

11. Sehnenreparaturvorrichtung nach Anspruch 10, wobei die chirurgische Schnur eine Naht ist, gegebenenfalls wobei die Naht mit Widerhaken versehen ist.

12. Sehnenreparaturgerüst nach einem der Ansprüche 1 bis 9 zur Verwendung in einem Verfahren zur Behandlung des menschlichen oder tierischen Körpers, wobei das Verfahren das Behandeln einer beschädigten Sehne umfasst.

13. Verfahren zum Herstellen eines Sehnenreparaturgerüsts nach einem der Ansprüche 1 bis 9, wobei das Verfahren die folgenden Schritte umfasst:
(A) Elektrospinnen einer Vielzahl von Fasern, die PCL umfassen;
(B) Bilden eines Garns durch Ausrichten und Verdrehen der Vielzahl von Fasern; und
(C) Stricken des Garns unter Verwendung eines Schussstrickverfahrens, um einen gestrickten Körper zu bilden, der eine Vielzahl von Öffnungen umfasst.

14. Verfahren nach Anspruch 13, wobei das Garn mit einem Drei-Nadel-Schussstrickverfahren gestrickt wird.

## Revendications

1. Échafaudage de réparation de tendon comprenant un corps tricoté, dans lequel
le corps tricoté est constitué d'un fil comprenant de la polycaprolactone (PCL) ;
le corps tricoté comprend une pluralité d'ouvertures ;
les ouvertures sont ouvertes au niveau et entre une première configuration dans laquelle le corps tricoté est déchargé et présente une première longueur, et une deuxième configuration dans laquelle le corps tricoté est chargé et présente une deuxième longueur qui est au moins 10 % supérieure à la première longueur ; et
le corps tricoté est formé en tricotant le fil en utilisant un procédé de tricotage de trame.

2. Échafaudage de réparation de tendon selon la revendication 1, dans lequel le fil comprend une pluralité de fibres comprenant de la PCL, facultativement dans lequel les fibres sont réalisées par électrofilage et/ou dans lequel la pluralité de fibres sont alignées et/ou dans lequel le fil est formé par torsion de la pluralité de fibres.

3. Échafaudage de réparation de tendon selon la revendication 2, dans lequel le diamètre moyen des fibres est inférieur à 1 µm, préférablement entre 400 nm et 850 nm.

4. Échafaudage de réparation de tendon selon l'une quelconque des revendications précédentes, dans lequel le diamètre moyen du fil est inférieur à 500 µm, préférablement entre 225 µm et 275 µm.

5. Échafaudage de réparation de tendon selon l'une quelconque des revendications précédentes, dans lequel la première longueur du corps tricoté est comprise entre 15 mm et 25 mm.

6. Échafaudage de réparation de tendon selon l'une quelconque des revendications précédentes, dans lequel la largeur moyenne du corps tricoté dans la première configuration est comprise entre 1,5 mm et 2,5 mm.

7. Échafaudage de réparation de tendon selon l'une quelconque des revendications précédentes, dans lequel les ouvertures présentent individuellement une surface d'au moins 10 000 µm² dans la première configuration et une surface d'au moins 10 000 µm² dans la deuxième configuration.

8. Échafaudage de réparation de tendon selon l'une quelconque des revendications précédentes, comprenant un premier prolongement s'étendant à partir d'une première extrémité du corps tricoté, comprenant facultativement un deuxième prolongement s'étendant à partir d'une deuxième extrémité du corps tricoté.

9. Échafaudage de réparation de tendon selon la revendication 8, dans lequel le premier prolongement et, le cas échéant, le deuxième prolongement sont réalisés dans le même fil que le corps tricoté.

10. Dispositif de réparation de tendon comprenant un cordon chirurgical fixé à l'échafaudage de réparation de tendon selon l'une quelconque des revendications précédentes.

11. Dispositif de réparation de tendon selon la revendication 10, dans lequel le cordon chirurgical est une suture, facultativement dans lequel la suture est barbelée.

12. Échafaudage de réparation de tendon selon l'une quelconque des revendications 1 à 9 pour une utilisation dans un procédé de traitement du corps humain ou animal, dans lequel le procédé comprend le traitement d'un tendon endommagé.

13. Procédé de réalisation d'un échafaudage de réparation de tendon selon l'une quelconque des revendications 1 à 9, le procédé comprenant les étapes suivantes :
(A) l'électrofilage d'une pluralité de fibres comprenant de la PCL ;
(B) le formage d'un fil par alignement et torsion de la pluralité de fibres ; et
(C) le tricotage du fil en utilisant un procédé de tricotage de trame pour former un corps tricoté comprenant une pluralité d'ouvertures.

14. Procédé selon la revendication 13, dans lequel le fil est tricoté en utilisant un procédé de tricotage de trame à trois aiguilles.
